# EUROPEAN PATENT APPLICATION

(11) **EP 0 884 046 A1**
(43) Date of publication of application: **16.12.1998**
(21) Application number: 97201625.7
(22) Date of filing: 30.05.1997
(51) Int. Cl.: A61K 7/48

(54) **Cosmetic composition with photoprotective properties**

(71) Applicant: Sara Lee/DE N.V., 3532 AA Utrecht (NL)
(72) Inventor: Eckart, Karl-Heinz Voss, 51375 Leverkusen (DE); Finkel, Peter, 51519 Odenthal (DE)
(74) Representative: Smulders, Theodorus A.H.J., Ir.

(57) **Abstract**

The invention is directed to a cosmetic composition with synergistically improved photoprotective properties, comprising
- at least one component selected from the group of Vitamin E, Vitamin C, derivatives of Vitamin E, derivatives of Vitamin C, and combinations of at least two of these components, and
- at least one natural polyamine or derivative.

## Description

The invention is directed to cosmetic compositions with photoprotective properties, more in particular to compositions providing protection against UV-radiation from the sun (suncreams and the like)

UV exposure of the skin (UV-A to a greater extent than UV-B) leads to the generation of various free oxygen radicals in the skin. These free radicals attack in an entirely unselective manner precisely those skin constituents which are responsible for retention of elasticity and of moisture in the skin. Accordingly, they are starting point for several photochemical chain reactions, which finally may promote or lead to photo-induced skin damages like sunburn, skin ageing, immuno-suppression and possibly skin cancer.

Even if the skin is protected by UV-absorbing or - reflecting substances such as UV filters, pigments and micropigments, some UV radiation penetrates into the skin, which is quite often sufficient to start the above mentioned photochemical processes.

In order to provide a protection of the skin against the formation of these free radicals, skin-care compositions are provided on the skin which contain anti-oxidants that may penetrate the skin and act as radical scavengers.

Among the most effective radical scavengers are the Vitamin C and Vitamin E based derivatives. They may be used on the skin in various formulations, for example such as described in EP-A 579078. According to this publication the scavenger is used in combination with phytantriol, a compound enhancing the skin penetration.

However, there is a need for compositions having an improved effectivity as radical scavenger, either resulting in the use of smaller amounts of active components to obtain the same result, or in compositions having improved effectivity as photoprotective skin-care composition.

The invention is based on the surprising discovery, that naturally occurring polyamines, more in particular polyamines occurring in human or animal tissue (such as skin), in combination with Vitamin E and C, respectively derivatives thereof, show a synergistic improvement in effectivity.

Accordingly the invention is directed to a cosmetic composition with photoprotective properties, comprising
- at least one component selected from the group of Vitamin E, Vitamin C, derivatives of Vitamin E, derivatives of Vitamin C, and combinations of at least two of these components, and
- at least one natural polyamine or derivative (such as a salt or an acid addition salt).

Surprisingly it has been found that a combination very small amounts of these polyamines (about 50 ppm) with one or more of these Vitamins, provides a protection that is at least one order of magnitude larger than the protection of the Vitamin alone.

The naturally occurring polyamine is preferably a three- or tetra-amine, which contain at least one primary and at least one secondary amine group; more in particular it has the formula

HNR-(CH₂)ₘ-NH-(CH₂)ₙ-NH₂

wherein R denotes H or (CH₂)ₖNH₂, wherein k, m and n are 2 or more, and the sum thereof does not exceed 10. Especially preferred compounds are spermine (m = 4, k = 3 and n = 3) and spermidine (R = H, m = 4 and n = 3).

The polyamine is present in an amount of 10 ppm, calculated on the weight of the composition, preferably in an amount of between 10 en 500 ppm.

The amounts of Vitamins to be used in the composition are preferably for Vitamin C or derivative thereof at least 0.005 wt.%, preferably between 0.005 and 1 wt.%, and for Vitamin E or derivative thereof at least 0.01 wt.%, preferably between 0.01 and 2.5 wt.%.

The composition according to the invention preferably contains both one component selected from Vitamin C and derivatives thereof, and one from Vitamin E and derivatives thereof. Such a composition provides an even better protection than systems based on polyamine with one Vitamin only.

Suitable derivatives of the Vitamins are salts and esters, such as the alkali and alkaline earth metal salts (sodium, potassium, calcium, magnesium and the like) and the phosphate, linoleate or acetate.

The composition further contains various conventional ingredients for photoprotective cosmetic compositions. Depending on the type of composition, body milk, suncream, sun lotion and the like various types of additives may be present.

As indicated above the use of phytantriol (2-(dihydroxyethyl)-2-hydroxy-6,10,14-trimethyl-pentadecane) or another vehicle for enhancing skin penetration may be contemplated. Suitable amounts will range from 0.05 to 20 wt.%, depending on the type of application.

The compositions generally contain at least one UV-filter in a preferred amount of between 0.05 to 15 wt.%. Suitable as UV filter are each and every UV-absorbing compounds mentioned in the EC positive list and which are published in the 14^{th} Guideline 92/8/EEC of the European Commission, dated 18-2-92.

These filters are generally benzylidenecamphor compounds, p-aminobenzoic acid and derivatives thereof; benzophenone derivatives and benzotriazole derivatives.

The following compounds are preferably employed as UV filters:
N-propoxylated ethyl 4-aminobenzoate (mixture of isomers) ethoxylated ethyl 4-aminobenzoate
glyceryl 4-aminobenzoate
2-ethylhexyl-4-dimethylaminobenzoate
2-ethylhexyl salicyclate
isopentyl 4-methoxycinnamate (mixture of isomers)
2-ethylhexyl 4-methoxycinnamate
2-hydroxy-4-methoxy-4'-methyl-benzophenone [mexenone (INN)]
2-hydroxy-4-methoxybenzophenone-5-sulphonic acid and sodium salt (sulisobenzone and sodium salt),
α-(2-oxoborn-3-ylidene-toluene)-4-sulphonic acid and salts thereof
3-(4'-methylbenzylidene)-d,1-camphor
3-benzylidenecamphor
4-isopropyl-dibenzoylmethane
4-isopropylbenzyl salicyclate
1-(4-tert-butylphenyl)-3-(4-methoxyphenyl)propane-1,3-dione
2,4,6-trianilin-(p-carbo-2'-ethylhexyl-1'-oxy)-1,3,5-triazine.

The following compounds are particularly preferably used as UV filters:
2-ethoxyhexyl p-(dimethylamino)-benzoate;
2-ethylhexyl p-methoxycinnamate;
3-(4'-methylbenzylidene)-d,1-camphor;
2-hydroxy-5-methoxybenzophenone;
2-hydroxy-4-methoxybenzophenone-5-sulphonic acid;
2-phenylbenzimidazole-5-sulphonic acid.

Along with the above-mentioned combination of active compounds, the cosmetic product according to the invention contains bases and auxiliaries which are conventionally employed in cosmetics, in particular stabilisers and antioxidants such as butylhydroxyanisole, butylhydroxytoluene, EDTA salts such as magnesium sulphate, in amounts from 0.02 to 5%, inter alia.

The bases and auxiliaries additionally include solvents which are customary in cosmetics, such as water to 80%, monoalcohols, lower polyalcohols having 1 to 6 carbon atoms or mixtures of these, furthermore fatty material, such as mineral, animal, or vegetable oils such as paraffin oil, or waxes such as microwax, fatty acids, fatty alcohols, fatty acid esters such as cetylstearyl isononanoate and isopropyl palmitate, fatty alcohol ethers, oxyethylated fatty alcohols, lanolin and derivatives, as well as silicone oils in amounts from 0.5 to 50%, preferably 0.5 to 30%, particularly preferably in amounts from 5 to 30%.

If appropriate, the cosmetic skin-care product according to the invention contains emulsifiers in amounts from 0.1 to 20%, preferably in amounts from 0.2 to 10%, these emulsifiers being emulsifiers conventionally employed in cosmetics, in particular non-ionic, anionic, cationic or amphoteric compounds, for example sterols, polyol fatty acid esters and polyol fatty alcohol ethers, alkali metal salts and triethanolamine salts of fatty acid, sodium cetylstearyl sulphate, tetracylammonium halides and phospholipids. Examples of these are glycerol sorbitan fatty acid esters, polyoxyethylene fatty acid esters and alkyltetraglycol ether o-phosphoric acid esters.

0.02 to 5%, preferably 0.1 to 2%, of thickeners and gelling agents can furthermore be employed in the product according to the invention. These include polyacrylic acid derivatives, cellulose derivatives, bentonites, xanthan derivatives, alginates, guar gum and locust bean gum Polyacrylamide and zinc stearate are examples.

The preparation according to the invention can contain other substances which are customary in cosmetics. These include humectants (0.5 to 15%), colorants, buffer substances, preservatives and perfume oils in amounts from 0.01 and 5.0%.

The following may be mentioned by way of example as humectants: lower polyalcohols such as glycerol, propylene glycol, butylene glycol, sorbitol, moreover 2-pyrrolidone-5-carboxylic acid and the sodium salt thereof, lactic acid and the salts thereof, urea, proteins and protein derivatives such as collagent, and furthermore hyaluronic acid, inter alia.

Colorants to be added to the cosmetic preparations according to the invention which may be mentioned by way of example are:

Colour C.I. 16255, colour C.I. 61570, colour C.I. 42051, colour C.I. 15985, colour C.I. 77492. The following are preferably suitable as preservatives:
2,4-hexadienoic acid (sorbic acid and salts thereof),
4-hydroxybenzoic acid, salts and esters thereof,
3-acetyl-6-methyl-2,4(3H)-pyrandione (dehydracetic acid) and salts thereof,
1,1-methylene-bis-(3-(1-hydroxymethyl-2,4-dioximidazolidin-5-yl)-urea),
imidazolindinylurea,
2-phenoxy-ethanol,
benzyl alcohol.

The cosmetic skin-care product according to the invention is preferably in the form of an emulsion (cream or milk), such as oil-in-water or water-in-oil emulsions. It is generally prepared by mixing and stirring of the components, if appropriate followed by homogenisation, and if appropriate and preferably in an evacuated apparatus.

All percentages in the present text relate to percentages by weight, unless stated otherwise.

The invention is illustrated hereinafter in greater detail with the aid of the examples, without this being intended to have a restrictive character.

### EXAMPLE 1

By the use of the in-vivo β-carotin model:
- dermal application of the test product
- waiting period ensuring penetration of the formulation into skin
- treatment with β-carotin
- irradiation of the skin with UV light containing sufficient UV-A (test area and untreated control)
- compare photochemical degradation of β-carotin by UV-induced radicals of treated and untreated skin

To achieve a sufficient protection level about 0.6 % Vitamin E or 0.1 % of Vitamin C had to be used. In combination with 50 ppm spermine the level of Vitamin C could be 0.05 % and of Vitamin E 0.1 %, for obtaining the same level of activity.

A combination of 50 to 100 ppm spermine, 0.05 % Vitamin E and 0.01 % Vitamin C also provided sufficient protection.

### EXAMPLE 2

A sun protection balm was prepared from:

| | |
|---|---|
| Glycerol (waterfree) | 3.0 |
| D-panthenol | 1.0 |
| Xanthan gum | 0.1 |
| carbomer | 0.5 |
| 45 % NaOH | 0.45 |
| Vitamin C phosphate | |
| Sodium salt | 0.1 |
| Spermine | 0.025 |
| 96% ethanol | 8.0 |
| Isopropylpalmitate | 6.0 |
| phenyl trimethicone | 3.0 |
| Tocopherolacetate | 1.0 |
| Phytantriol | 0.1 |
| Perfume | 0.5 |
| Water | 76.225 |

## Claims

1. Cosmetic composition with photoprotective properties, comprising
- at least one component selected from the group of Vitamin E, Vitamin C, derivatives of Vitamin E, derivatives of Vitamin C, and combinations of at least two of these components, and
- at least one natural polyamine or derivative.

2. Composition according to claim 1, wherein the composition additionally contains UV-absorbing and/or reflecting compounds.

3. Composition according to claim 1 or 2, wherein the composition additionally contains one or conventional ingredients for cosmetic photoprotective compositions.

4. Composition according to claim 1-3, wherein the composition contains a polyamine naturally occurring in human or animal tissue, as the natural polyamine.

5. Composition according to claim 4, wherein the natural polyamine is a three- or tetra-amine, which contain at least one primary and at least one secondary amine group.

6. Composition according to claim 5, wherein the polyamine has the formula
HNR-(CH₂)ₘ-NH-(CH₂)ₙ-NH₂
wherein R denotes H or (CH₂)ₖNH₂, wherein k, m and n are 2 or more, and the sum thereof does not exceed 10.

7. Composition according to claim 1-6, wherein the natural polyamine is present in an amount of at least 10 ppm, calculated on the weight of the composition, preferably in an amount of between 10 en 500 ppm.

8. Composition according to claim 1-7, wherein a combination of at least one of Vitamin E or derivative of Vitamin E, and Vitamin C or, derivative of Vitamin C, is present.

9. Composition according to claim 1-8, wherein the amount of Vitamin C or derivative thereof is at least 0.005 wt.%, preferably between 0.005 and 1 wt.%, and the amount of Vitamin E or derivative thereof is at least 0.01 wt.%, preferably between 0.01 and 2.5 wt.%.

10. Composition according to claim 1-9, wherein the derivatives of Vitamin E and Vitamin C are selected from salts and esters thereof.

11. Composition according to claim 1-10, in the from of a sun lotion, body milk or suncream.

12. Use of a combination of a natural polyamine and at least one of Vitamin E, Vitamin C, derivatives of Vitamin E and derivatives of Vitamin C, for synergistically improving the UV-protection level in cosmetic compositions.
